(19) Europäisches Patentamt
European Patent Office
Office européen des brevets

(11) Publication number: 0 171 832 B1

(12) EUROPEAN PATENT SPECIFICATION

(45) Date of publication of patent specification: 06.09.89

(21) Application number: 85201075.0

(22) Date of filing: 03.07.85

(51) Int. Cl.$^4$: C 07 D 317/72, C 07 D 303/04, C 07 D 303/08, C 07 D 303/34, C 07 D 303/36, C 07 D 303/14, C 07 D 303/22, C 07 C 31/20, C 07 C 31/22, C 07 C 31/42, C 07 C 43/13

# (54) Camphor derivatives, their preparation and their use.

(30) Priority: 19.07.84 GB 8418404

(43) Date of publication of application: 19.02.86 Bulletin 86/08

(45) Publication of the grant of the patent: 06.09.89 Bulletin 89/36

(84) Designated Contracting States: BE CH DE FR GB IT LI NL SE

(56) References cited:
US-A-3 491 152

JOURNAL OF THE CHEMICAL SOCIETY, CHEMICAL COMMUNICATIONS, 1984, pages 1600-1602, London, GB; M.K. ELLIS et al.: "Kinetic resolution of 1,2-diols with D-camphorquinone; preparation of (R)-(chloromethyl)oxirane"

(73) Proprietor: SHELL INTERNATIONALE RESEARCH MAATSCHAPPIJ B.V.
Carel van Bylandtlaan 30
NL-2596 HR Den Haag (NL)

(72) Inventor: Watson, William Peter
511 Maidstone Road
Rainham Kent ME8 0JX (GB)
Inventor: Golding, Bernard Thomas
2 Glendyne Close Jesmond Park West Jesmond
Newcastle upon Tyne Tyne and Wear NE77BU (GB)
Inventor: Ellis, Martin Keith
83 Park Lane
Pontefract West Yorkshire WF8 4QJ (GB)

(74) Representative: Aalbers, Onno et al
P.O. Box 302
NL-2501 CH The Hague (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 832 B1

**Description**

This application relates to certain novel camphor derivatives, to a process for their preparation and to their use in processes for the preparation of optically active compounds. Specifically, it relates to certain stereoisomers of the camphor derivatives, their stereospecific synthesis, and their application in stereoselective synthesis of e.g. substituted 2-propanols, 2,3-epoxypropanes and like chiral molecules, which are of interest in the synthesis of e.g. medicinal compounds.

It is known from J. Casanova and E. J. Corey, *Chem. Ind.* (1961) 1664 to ketalise D-(−)-butane-2,3-diol with *rac*-camphor, and to separate the roughly equimolar stereoisomer mixture by gas liquid chromatography. Analogously, the Applicants found that the reaction of either (*R*)- or (*S*)-propane-1,2-diol with D-camphor gave a 1:1 mixture of stereoisomeric ketals. Changing the reaction medium from refluxing benzene to refluxing toluene did not influence the proportion of the products formed. Apparently it is possible to react camphor or a stereoisomer thereof with a vicinal diol, but this ketalisation reaction is not stereoselective, and thus not of much use in the preparation of optically active compounds or intermediates.

Surprisingly it has now been found that when camphorquinone or a stereoisomer thereof is reacted with a vicinal diol or a stereoisomer thereof, a nonequimolar mixture of stereoisomeric ketals is formed, from which mixture the predominantly formed stereoisomer can easily be separated. Convenient conversions of these ketals into epoxypropanes or propanediols in which their chirality is retained, have also been found.

Accordingly, the invention provides novel camphor derivatives, characterized by having general formula I or its enantiomeric structure

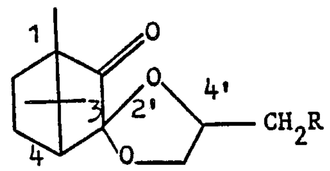

(I)

wherein R represents a hydrogen or halogen atom, a cyano or isocyanato group, a hydroxy group, an amino group or an optionally substituted alkyl, alkoxy, aryloxy or organic acyl group, which optionally substituted organic acyl group is a derivative of an optionally substituted carboxylic, phosphonic, phosphinic, sulphonic, sulphinic or sulphenic acid, and the optional substituents being selected from lower alkyl, lower alkoxy, phenyl and halogen. The organic acyl group is especially a derivative of a sulphonic acid. The halogen atom may be fluorine, chlorine, bromine or iodine. Chlorine and bromine are preferred, particularly chlorine. Optional substituents in the above groups are especially $CH_3$, $OCH_3$ and halogen.

R preferably represents a hydrogen or halogen atom or a hydroxy, $C_{1-4}$ alkoxy, phenoxy, naphthoxy, tosyl or mesyl group, optionally substituted by $CH_3$, $OCH_3$ or halogen, and R particularly represents a chlorine atom or a hydroxy group. Most preferably R is chlorine, because of the structural relationship to epichlorohydrin.

The molecule of the camphor derivative of the invention contains four chiral carbon atoms (disregarding any such atoms in the substituent R), but the configuration of one bridgehead carbon atom of the camphor-skeleton (the bicyclo[2.2.1]heptane frame) is determined by that of the other bridgehead carbon atom, with the result that there are eight possible isomers, which are drawn out in Figures Ia—h of the accompanying drawings. The absolute configurations can be tabulated as follows, assuming the group R takes priority over oxygen in determining the chirality (*R* or *S*) of the 4′-carbon atom:

| | |
|---|---|
| Ia: | 1*R* 3*R* 4*S* 4′*R* |
| Ib: | 1*R* 3*R* 4*S* 4′*S* |
| Ic: | 1*R* 3*S* 4*S* 4′*S* |
| Id: | 1*R* 3*S* 4*S* 4′*R* |
| Ie: | 1*S* 3*S* 4*R* 4′*S* |
| If: | 1*S* 3*S* 4*R* 4′*R* |
| Ig: | 1*S* 3*R* 4*R* 4′*R* |
| Ih: | 1*S* 3*R* 4*R* 4′*S* |

Should the substituent R not take priority over oxygen, for example if it was a hydrogen atom, then all (4′*S*) become (4′*R*) and vice versa. The rules for determining *R* or *S*-chirality are those of Cahn, Ingold and

Prelog, *Angew. Chem. intern. Edit.*, *5* (1966) 385 (and errata on p. 511), as adopted by the International Union for Pure and Applied Chemistry in their Nomenclature of Organic Chemistry, Section E (Stereochemistry).

The present invention comprises individual stereoisomers of the compounds of formula I, and mixtures thereof. Preferred stereoisomers are those of Figures Ia and Ie, as these are of the greatest technical interest as intermediates in the preparation of more complex optically active molecules.

In another aspect the invention provides a process for the preparation of camphor derivatives as defined above, characterised in that a 1,2-diol of formula $CH_2OH—CHOH—CH_2R$, wherein R is as defined above, is reacted with camphorquinone in the presence of an acidic catalyst.

Camphorquinone (1,7,7-trimethylbicyclo[2.2.1]heptane-2,3-dione) is a known compound and can be prepared by oxidation of camphor. When starting from an optical isomer of camphor, the chirality can be retained, using for instance the method of Evans, Ridgion and Simonsen, *J. Chem. Soc.* (1934) 137. Camphor itself is a natural product and is also made on a large scale from alpha-pinene (turpentine).

Surprisingly only one keto group of the quinone is ketalised under the conditions of the reaction.

The reaction with the 1,2-diol is essentially a ketalisation, and is performed in the manner known in the art for such reactions. A catalytic amount of acid, either in homogeneous form, e.g. anhydrous HCl, or in heterogeneous form, e.g. an acidic ion-exchange resin, is essential. Conveniently a minor amount of a strong organic acid, e.g. p-toluene-sulphonic acid, is present. The molar ratios of the reactants may be in the range of 1,2-diol:camphorquinone = 1:1 to 50:1, but preferably a small excess of the diol is used. The preferred molar ratios thus are in the range of 2:1 to 10:1. The reaction may be carried out in the absence of solvents or diluents when one or both reactants are liquid, but preferably a solvent is employed. Suitable inert solvents are apolar aprotic organic liquids such as $CCl_4$, toluene and, especially, benzene.

In a preferred embodiment of the invention, the stereoselective properties of the reaction are employed to prepare one or more specific stereoisomers of the compound. One could start from D- or L-camphorquinone and/or from a (2*R*)- or (2*S*)-1,2-diol. The camphor enantiomers, and therefore the camphorquinone enantiomers, are readily available, and it is therefore advantageous to react the (racemic)-1,2-diol with either D- or L-camphorquinone to yield a mixture of stereoisomers of camphor derivatives of formula I, in which the main component comprises the stereoisomer of the configuration of either Figure Ia or Figure Ie, respectively. Nevertheless, both (*R*)- or (*S*)-propane-1,2-diol have been made, and could be used too, by way of illustrating a synthesis with one isomer of the 1,2-diol. The main component of the mixture (formed by the reaction between D- or L-camphorquinone and the *rac*-1,2-diol) is suitably prepared by subjecting the mixture to a physical separation process. This physical separation process advantageously comprises fractional crystallisation or chromatography. By employing a special kind of chromatography such as preparative high performance liquid chromatography (HPLC), one can actually separate all stereoisomers formed; however, often one would be interested in the main product only and a single fractional crystallisation step would be sufficient.

It has been found that at higher reaction temperatures the ratios of stereoisomers formed differ from those at lower temperatures. The lower the reaction temperature, the more the stereoisomer distribution is governed kinetically instead of by thermodynamic energy differences. Especially when it is desired to prepare one or more stereoisomers in preference to others, it is advantageous to work at lower temperatures. Preferably the reaction is carried out at a temperature below 90°C, especially below 80°C, but not below a temperature at which the reaction time becomes excessive, i.e. preferably not below 20°C, more preferably not below 40°C.

As stated hereinbefore, one use of the compounds of the invention resides in their being valuable starting materials for the preparation of a number of "natural" products and pharmaceutical, pesticidal or olfactory compounds. The compounds of the invention may also advantageously be converted in other compounds of synthetic interest, such as (chiral) epoxypropanes or (chiral) 1,2-diols. For example, it is possible, (see J. J. Baldwin *et al., J. Org. Chem.* Vol. 43 No. 25, 1978, pp. 4876 to 4878) to synthesize (*R*)-recifeiolide from (*R*)-methyloxirane, or to prepare (*S,S*)-vermiculin from (*S*)-(2-bromoethyl)oxirane, which starting materials can be made from the present compounds wherein R is hydrogen and $CH_2Br$, respectively. Other syntheses, e.g. using (R)-epichlorohydrin ((R)-chloromethyloxirane) are illustrated in U.S. Patent 4,408,063.

Accordingly, the invention also relates to a process for the preparation of a (2*R*)- or a (2*S*)-1,2-epoxypropane of formula

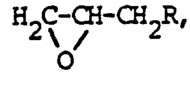

which includes a process as defined above, characterised in that the camphor derivative(s) prepared is (are) further reacted with a mixture of AcOH and HX (wherein Ac is an acyl group derived from a strong organic acid and X stands for Cl, Br or I), followed by a cyclization reaction using a basic reagent. The chirality of the relevant carbon atoms is retained, and thus (2*R*)-1,2-epoxypropane is prepared from the (4'*R*)-stereoisomer(s) of formula I, and analogously the (2*S*)-1,2-epoxypropane is prepared from the (4'*S*)-stereoisomer(s).

The first reaction, suitably with HCl or HBr dissolved in acetic acid, yields a mixture of camphorquinone and an acyloxypropane of formula $CH_2X—CH(OAc)—CH_2R$. The acid HX should be chosen such that X is

not equal to R, because otherwse an optically inactive (intermediate) compound would be prepared. The acyloxypropane may be isolated from the product mixture, but conveniently the mixture is treated as such with a base to effect a cyclization to the methyl-oxirane (epoxypropane). The base may be any organic or inorganic acid-binding material, for example NaOH, $NaOCH_3$, n-butyllithium, but preferably use is made of sodium ethylene glycolate ($NaOCH_2CH_2OH$, conveniently in the form of a mixture of $NaOCH_2CH_2ONa$ and $HOCH_2CH_2OH$) because of the easier work-up it allows. Thus, the epoxypropane is preferably prepared by a process as defined, in which the mixture of AcOH and HX comprises HBr in acetic acid and the basic reagent comprises $NaOCH_2CH_2OH$.

It is known from J. J. Baldwin et al., *J. Org. Chem.*, Vol. 43, No. 25, 1978, pp. 4876—8, that (R)- and (S)-epichlorohydrin may be prepared in eight or six steps, respectively, from the bis-acetonide of optically active D-mannitol. The present process thus provides a substantially easier method of preparing (*R*)- and/or (*S*)-epichlorohydrin, in addition to being applicable to all kinds of substituents R containing compounds.

In some cases it may be desirable to prepare propanediols with certain substituents R, especially (2*R*)- or (2*S*)-stereoisomers thereof, which is also made very easy by the present invention. Accordingly the invention also relates to a process for the preparation of a (2*R*)- or a (2*S*)-1,2-diol of formula $CH_2OH—CHOH—CH_2R$, which includes a process as defined above, characterised in that the camphor derivative(s) prepared is (are) further reacted with a selective reduction agent, followed by hydrolyzing or alcoholysing the corresponding camphol derivative thus formed. It will be evident that where the compounds of the invention can be prepared from *rac*-1,2-diol of formula $CH_2OH—CHOH—CH_2R$, the present process provides a chemical resolution of the enantiomers of this diol (of course R should not be OH).

The selective reduction agent reduces the keto group of the camphor-moiety of the compounds of the invention to an alcohol group, thus making a camphol derivative. The chirality of each molecule is retained. Suitable reduction agents are the alkali metal-metal hydride compounds, such as $LiAlH_4$ or $NaBH_4$, but gaseous hydrogen with a suitable catalyst (Pd, Pt, Ni or the like) could be used too. The camphol derivative is then de-ketalised with, preferably an excess of, water or an alcohol, suitably in the presence of acid or acidic catalyst, yielding a partially reduced camphorquinone (possibly ketalised by the other alcohol) and the desired 1,2-diol stereoisomer. Preferably the reduction agent is $NaBH_4$ and the camphol derivative is preferably treated with an acidified lower alkanol, especially methanol or ethanol acidified with a stong mineral acid such as HCl.

The invention is further illustrated by the following examples. All compounds were analyzed physically by infrared spectroscopy, electron impact mass spectrometry, $^1H$ and $^{13}C$ nuclear magnetic resonance, as well as chemically, confirming the assigned structures.

## Examples

1. Preparation of (1*R* 3*R* 4*S* 4′*R*)-camphor-3-spiro-2′-(4′-chloromethyl-1′,3′-dioxolane) and 3 stereoisomers thereof

A stirred solution of D-camphorquinone (100 g, 0.6 mol), (*rac*)-3-chloropropane-1,2-diol (314 g, 238 $cm^3$, 2.86 mol) and p-toluenesulphonic acid (10 g, 0.052 mol) in benzene (1 750 $cm^3$) was boiled at 84°C for 18 h in a flask connected to a Dean-Stark apparatus. The solvent was removed (rotary evaporator) to give a yellow liquid that was covered with water (1000 $cm^3$). The resulting aqueous mixture was extracted with petrol (b.p. 40—60°C, 3 × 500 $cm^3$). The combined extracts were dried ($MgSO_4$) and evaporated to yield a yellow oil. This was dissolved in petrol-ethyl acetate (7:1) and was filtered through silica gel to give a colourless oily mixture of ketals (of the configuration of Figures Ia, Ib, Ic and Id wherein R = Cl; yields 45, 17, 27 and 12%, respectively, determined by hplc). The mixture crystallised on standing and was recrystallised repeatedly from petrol (b.p. 40—60°C) to afford the title compound (ketal Ia) as a colourless crystalline solid (14.6 g, 9.4%), m.p. 75.5—76.5°C (starts to sublime at 61°C), $[\alpha]_D^{20}$ +74° (c 1.5 in $CHCl_3$).

In a second experiment, the mixture of ketals was separated in one pass through a preparative hplc column [Whatman Partisil M20:10/50 (50 cm × 22 mm i.d.), elution with 2% (v/v) ethylacetate in hexane].

When ketal (Ia) was heated in toluene (103°C/96 h) containing a catalytic quantity of p-toluenesulphonic acid it equilibrated with ketal (Id) (ratio = 1:1 at equilibrium). Thus ketals Ia and Id have the same chirality at the carbon atom (4′) bearing a chloromethyl group, for only the spirocarbon atom is affected by the racemization. The structural assignments made for the ketals are additionally based on careful analysis of their 400 MHz $^1H$ nmr spectra.

2. Preparation of (*R*)-3-chloropropane-1,2-diol

The ketal prepared in Example 1 ((1*R* 3*R* 4*S* 4′*R*)-camphor-3-spiro-2′-(4′-chloromethyl-1′,3′-dioxolane)) (0.5 g, 1.9 × $10^{-3}$ mol) was mixed with $NaBH_4$ (0.14 g) in ethanol (10 ml). The mixture was heated at 45—50°C for 4 h under nitrogen. The solvent was removed and water was added to the residual oil. The resulting aqueous solution was extracted with diethyl ether (3 × 25 $cm^3$). The combined extracts were dried ($MgSO_4$) and the ether was removed to give a colourless oil. This was taken up in a mixture of 2 M hydrochloric acid (3 $cm^3$) and methanol (sufficient to give a homogeneous solution). The solution was boiled under reflux for 3 h. The solvents were removed and water (20 $cm^3$) was added. The resulting aqueous layer was evaporated and the residual yellow oil was taken up in dichloromethane. After drying ($MgSO_4$), the dichloromethane was removed to give an oil that was distilled (Kugelrohr):b.p. 75°C at 15

mmHg (115 mg, 55%), $[\alpha]_D^{19}$ −7.4°.

3. Preparation of (*R*)-chloromethyloxirane

The ketal prepared in Example 1 (3.0 g, 1.1 × $10^{-2}$ mol) was stirred in 9.4 ml of a solution of HBr in acetic acid (48% w/v) at 60°C for 5 hours. A mixture of camphorquinone and (*S*)-2-acetoxy-1-bromo-3-chloropropane was formed, as indicated by $^1$H nmr. To this mixture a solution of 1.2 M sodium ethane-1,2-diolate (9.16 ml, 1.1 × $10^{-2}$ mol) in ethane-1,2-diol was added and reacted for 15 min at 20°C. The title compound was distilled directly from the mixture: yield 0.6 g (58% based on starting ketal). The epoxide was chemically and optionally pure by $^1$H nmr spectroscopy (no enantiomer detected after addition of the chiral shift reagent tris[3-(heptafluoropropylhydroxymethylene)-d-camphorato]europium (III) and gave $[\alpha]_D^{24}$ −33° (C 1.5 in $CH_3OH$), which is comparable to the highest literature values [−34.3° for (*R*)-isomer, +33° (C 1.126 in $CH_3OH$) for (*S*)-isomers.

4. Preparation of (1*S* 3*S* 4*R* 4′*S*)-camphor-3-spiro-2′-(4′-chloromethyl-1′,3′-dioxolane)

The procedure of Example 1 was repeated with L-camphorquinone instead of the D-enantiomer. The title compound was isolated in 11.4% yield as a colourless crystalline solid, m.p. 74—76°C, $[\alpha]_D^{20}$ −71° (C 1.5 in $CHCl_3$).

5. Preparation of (1*R* 3*R* 4*S* 4′*S*)-camphor-3-spiro-2′-(4′-methyl-1′,3′-dioxolane and stereoisomers

(a) Example 1 was repeated with (*S*)-propane-1,2-diol instead of *rac*-3-chloropropane-1,2-diol. A 4:1 mixture of diastereoisomeric ketals (the title compound and the (1*R* 3*S* 4*S* 4′*S*) stereoisomer; configurations of figures Ia and Id, respectively) was formed, from which the predominant one could easily be crystallised: m.p. 35—37°C. When the latter, isolated compound was heated in toluene (105°C/96 h) it yielded a 2:3 mixture of the compounds of configurations Ia and Id.

(b) Example 1 was also repeated with (*R*)-propane-1,2-diol and D-camphorquinone, giving a 4:3 mixture of the ketals of configurations 1*R* 3*R*. 4*S* 4′*R*. and 1*R*3*S*. 4*S*4′*R*.

Evidently at the lower refluxing temperature of the benzene solvent the reaction is kinetically controlled, whereas at the higher temperature in toluene mixtures in the thermodynamic equilibrium ratios are formed.

The (*R*) and (*S*)-propane-1,2-diols were prepared as described in B. T. Golding, D. R. Hall and S. Sakrikar, J. Chem. Soc. Perkin Trans., 1 (1973) 1214 and P. A. Levene an A. Walti, Org. Synth. Coll., Vol II (1943) 5 and 545.

Comparative

Both (*R*)- and (*S*)-propane-1,2-diols, prepared as described in Example 5, were reacted with D-camphor in refluxing benzene or toluene in the presence of a catalytic quantity of p-toluenesulphonic acid. In all cases *ca* 1:1 mixtures of diastereoisomeric ketals were obtained. Apparently D-camphor does not possess the same stereospecific properties as D-camphorquinone.

## Claims

1. Camphor derivatives characterised by having general formula I or its enantiomeric structure

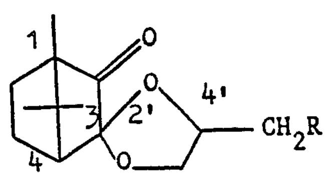

(I)

wherein R represents a hydrogen or halogen atom, a cyano or isocyanato group, a hydroxy group, an amino group, or an optionally substituted alkyl, alkoxy, aryloxy, or organic acyl group, which optionally substituted organic acyl group is a derivative of an optionally substituted carboxylic, phosphonic, phosphinic, sulphonic, sulphinic or sulphenic acid, and the optional substituents being selected from lower alkyl, lower alkoxy, phenyl and halogen.

2. A derivative according to claim 1, characterised in that R represents a hydrogen atom, a halogen atom, a hydroxy group or a $C_{1-4}$alkyl, hydroxy, $C_{1-4}$alkoxy, phenoxy, naphthoxy, tosyl or mesyl group optionally substituted by $CH_3$, $OCH_3$ or halogen.

3. A derivative according to claim 2, characterised in that R represents a chlorine atom or a hydroxy group.

4. A derivative according to any one of claims 1 to 3, characterised in that it has the configuration 1*R* 3*R* 4*S* 4′*R* or 1*S* 3*S* 4*R* 4′*S*.

5. Process for the preparation of camphor derivatives as claimed in claim 1, characterised in that a 1,2-diol of formula $CH_2OH—CHOH—CH_2R$, wherein R has the meaning defined in claim 1, is reacted with camphorquinone in the presence of an acidic catalyst.

6. A process according to claim 5, characterised in that the 1,2-diol is reacted with either D- or L-camphorquinone to yield a mixture of stereoisomers of camphor derivatives of formula I, in which the main component comprises the stereoisomer of the configuration 1*R* 3*R* 4*S* 4'*R* or 1*S* 3*S* 4*R* 4'*S*.

7. A process according to claim 6, characterised in that the main component of the mixture is prepared by subjecting the mixture to a physical separation process.

8. A process according to claim 7, characterised in that the physical separation comprises fractional crystallisation or chromatography.

9. A process according to any one of claims 5 to 8, characterised in that the reaction is carried out at a temperature below 90°C.

10. Process for the preparation of a (2*R*)- or a (2*S*)-1,2-epoxypropane of formula

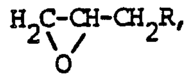

which includes a process according to any one of claims 5 to 9, characterised in that the camphor derivative(s) prepared is (are) further reacted with a mixture of AcOH and HX (wherein Ac is an acyl group derived from a strong organic acid and X stands for Cl, Br or I), followed by a cyclization reaction using a basic reagent.

11. A process according to claim 10, characterised in that the mixture of AcOH and HX comprises HBr in acetic acid, and the basic reagent comprises $NaOCH_2CH_2OH$.

12. Process for the preparation of a (2*R*)- or a (2*S*)-1,2-diol of formula $CH_2OH—CHOH—CH_2R$, which includes a process according to any one of claims 5 to 9, characterised in that the camphor derivative(s) prepared is (are) further reacted with a selective reduction agent, followed by hydrolyzing or alcoholyzing the corresponding camphol derivative thus formed.

13. A process according to claim 12, characterised in that the reduction agent is $NaBH_4$ and that the camphol derivative is treated with an acidified lower alkanol.

14. Use of a camphor derivative as claimed in claim 1 in a process for the preparation of optically active compounds of formula $CH_2OHCHOHCH_2R$ or formula

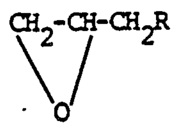

wherein R is as defined in Claim 1.

**Patentansprüche**

1. Campherderivate dadurch gekennzeichnet, daß sie die allgemeine Formel (I) besitzen oder deren enantiomere Struktur

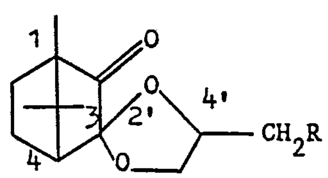

(I)

wobei R ein Wasserstoff- oder Halogenatom, eine Cyano- oder Isocyanatogruppe, eine Hydroxygruppe, eine Aminogruppe oder eine gegebenenfalls substituierte Alkyl-, Alkoxy-, Aryloxy- oder organische Acylgruppe bedeutet, wobei die gegebenenfalls substituierte organische Acylgruppe ein Derivat einer gegebenenfalls substituierten Carbon-, Phosphon-, Phosphin-, Sulfon-, Sulfin- oder Sulfensäure ist, und die gegebenenfalls vorhandenen Substituenten ausgewählt sind aus Nieder-Alkyl, Nieder-Alkoxy, Phenyl und Halogen.

2. Derivat nach Anspruch 1, dadurch gekennzeichnet, daß R ein Wasserstoffatom, ein Halogenatom, eine Hydroxygruppe oder eine $C_{1-4}$ Alkyl-, Hydroxy-, $C_{1-4}$ Alkoxy-, Phenoxy-, Naphthoxy-, Tosyl- oder Mesylgruppe, gegenbenenfalls substituiert durch $CH_3$, $OCH_3$ oder Halogen, bedeutet.

3. Derivat nach Anspruch 2, dadurch gekennzeichnet, daß R ein Chloratom oder eine Hydroxygruppe bedeutet.

4. Derivat nach einem der Ansprüche 1 bis 3, dadurch gekennzeichnet, daß es die Konfiguration 1*R* 3*R* 4*S* 4'*R* oder 1*S* 3*S* 4*R* 4'*S* besitzt.

5. Verfahren zur Herstellung von Campherderivaten nach Anspruch 1, dadurch gekennzeichnet, daß ein 1,2-Diol der Formel $CH_2OH—CHOH—CH_2R$, in der R die in Anspruch 1 angegebene Bedeutung hat, umgesetzt wird Campherchinon in Gegenwart eines sauren Katalysators.

6. Verfahren nach Anspruch 5, dadurch gekennzeichnet, daß das 1,2-Diol umgesetzt wird entweder mit D- oder L-Campherchinon unter Bildung eines Gemisches von Stereoisomeren von Campherderivaten der Formel (I), in dem die Hauptkomponente das Stereoisomer der Konfiguration 1*R* 3*R* 4*S* 4'*R* oder 1*S* 3*S* 4*R*

4'*S* umfaßt.

7. Verfahren nach Anspruch 6, dadurch gekennzeichnet, daß die Hauptkomponente des Gemisches hergestellt (isoliert) wird, indem man das Gemisch einem physikalischen Trennverfahren unterwirft.

8. Verfahren nach Anspruch 7, dadurch gekennzeichnet, daß die physikalische Trennung eine fraktionierte Kristallisation oder Chromatographie umfaßt.

9. Verfahren nach einem der Ansprüche 5 bis 8, dadurch gekennzeichnet, daß die Reaktion bei einer Temperatur unterhalb 90°C durchgeführt wird.

10. Verfahren zur Herstellung eines (2*R*)- oder eines (2*S*)-1,2-Epoxypropans der Formel

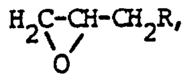

umfassend ein Verfahren nach einem der Ansprüche 5 bis 9, dadurch gekennzeichnet, daß das hergestellte Campherderivat (die Derivate) ferner umgesetzt wird (werden) mit einem Gemisch von AcOH und HX (wobei Ac eine Acylgruppe ist, die von einer starken organischen Säure abgeleitet ist und X Cl, Br oder I bedeutet), gefolgt von einer Cyclisierungsreaktion unter Verwendung eines basischen Reagenses.

11. Verfahren nach Anspruch 10, dadurch gekennzeichnet, daß das Gemisch von AcOH und HX HBr in Essigsäure umfaßt und das basische Reagens $NaOCH_2CH_2OH$.

12. Verfahren zur Herstellung eines (2*R*)- oder eines (2*S*)-1,2-Diols der Formel $CH_2OH—CHOH—CH_2R$, umfassend ein Verfahren nach einem der Ansprüche 5 bis 9, dadurch gekennzeichnet, daß das hergestellte Campherderivat (die Derivate) ferner umgesetzt wird (werden) mit einem selektiven Reduktionsmittel, gefolgt von einer Hydrolyse oder Alkoholyse des so gebildeten entsprechenden Campherderivats.

13. Verfahren nach Anspruch 12, dadurch gekennzeichnet, daß das Reduktionsmittel $NaBH_4$ ist und das Campherderivat mit einem angesäuerten niederen Alkanol behandelt wird.

14. Verwendung eines Campherderivats nach Anspruch 1 in einem Verfahren zur Herstellung von optisch aktiven Verbindungen der Formel $CH_2OHCHOHCH_2R$ oder der Formel

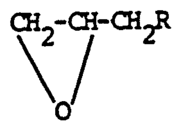

wobei R wie in Anspruch 1 definiert ist.

**Revendications**

1. Dérivés du camphre caractérisés en ce qu'ils ont la formule générale I ou sa structure énantiomère

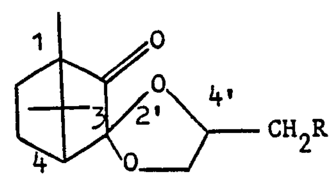

(I)

où R représente un atome d'hydrogène ou d'un halogène, un groupe cyano ou isocyanato, un groupe hydroxy, un groupe amino ou un groupe alcoyle, alcoxy, aryloxy ou acyle organique éventuellement substitué, le groupe acyle organique éventuellement substitué étant un dérivé d'un acide carboxylique, phosphonique, phosphinique, sulfonique, sulfinique ou sulfénique éventuellement substitué, et les substituants éventuels étant choisis parmi des groupes alcoyle inférieur, alcoxy inférieur, phényle et des halogènes.

2. Un dérivé selon la revendication 1, caractérisé en ce que R représente un atome d'hydrogène, un atome d'halogène, un groupe hydroxy ou un groupe $C_{1-4}$ alcoyle, hydroxy, $C_{1-4}$ alcoxy, phénoxy, naphtoxy, tosyle ou mésyle éventuellement substitué par $CH_3$, $OCH_3$ ou des halogènes.

3. Un dérivé selon la revendication 2, caractérisé en ce que R représente un atome de chlore ou un groupe hydroxy.

4. Un dérivé selon l'une quelconque des revendications 1 à 3, caractérisé en ce qu'il a la configuration 1*R* 3*R* 4*S* 4'*R* ou 1*S* 3*S* 4*R* 4'*S*.

5. Procédé pour la préparation de dérivés du camphre tels que revendiqués dans la revendication 1, caractérisé en ce qu'un 1,2-diol de formule $CH_2OH—CHOH—CH_2R$, où R a la signification définie dans la revendication 1, est mis à réagir avec de la camphoquinone en présence d'un catalyseur acide.

6. Un procédé selon la revendication 5, caractérisé en ce que le 1,2-diol est mis à réagir avec de la D- ou de la L-camphoquinone pour donner un mélange de stéreoisomères de dérivés du camphre de formule I, dans lequel le constituant principal comprend le stéréo-isomère de la configuration 1*R* 3*R* 4*S* 4'*R* ou 1*S* 3*S* 4*R* 4'*S*.

7. Un procédé selon la revendication 6, caractérisé en ce qu'on prépare le constituant principal du mélange en soumettant le mélange à une séparation physique.

8. Un procédé selon la revendication 7, caractérisé en ce que la séparation physique comprend une cristallisation fractionnée ou une chromatographie.

9. Un procédé selon l'une quelconque des revendications 5 à 8, caractérisé en ce que la réaction est conduite à une température au-dessous de 90°C.

10. Procédé pour la préparation d'un (2*R*)- ou d'un (2*S*)-1,2-époxypropane de formule

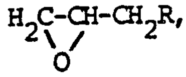

qui comprend un procédé selon l'une quelconque des revendications 5 à 9, caractérisé en ce que le ou les dérivés du camphre préparés sont encore mis à réagir avec un mélange de AcOH et de HX (où Ac est un groupe acyle dérivé d'un acide organique fort et X représente Cl, Br ou I), cela étant suivi d'une réaction de cyclisation utilisant un réactif basique.

11. Un procédé selon la revendication 10, caractérisé en ce que le mélange de AcOH et de HX comprend HBr dans l'acide acétique et que le réactif basique comprend $NaOCH_2CH_2OH$.

12. Procédé pour la préparation d'un (2*R*)- ou d'un (2*S*)-1,2-diol de formule $CH_2OH—CHOH—CH_2R$, qui comprend un procédé selon l'une quelconque des revendications 5 à 9, caractérisé en ce que le ou les dérivés du camphre préparés sont encore mis à réagir avec un agent reducteur sélectif, cela étant suivi d'une hydrolyse ou d'une alcoolyse du dérivé correspondant du camphre ainsi formé.

13. Un procédé selon la revendication 12, caractérisé en ce que l'agent réducteur est $NaBH_4$ et que le dérivé du camphre est traité par un alcanol inférieur acidifié.

14. Utilisation d'un dérivé du camphre tel que revendiqué dans la revendication 1 dans un procédé pour la préparation de composés optiquement actifs de formule $CH_2OHCHOHCH_2R$ ou de formule

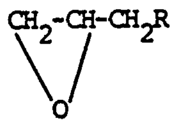

où R est tel que défini dans la revendication 1.

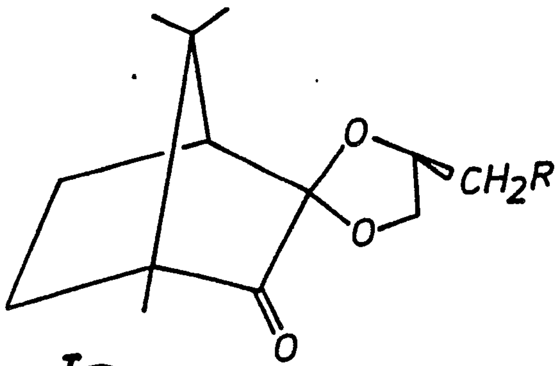

Ia

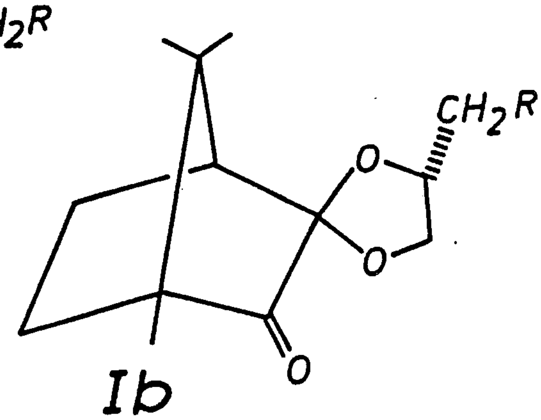

Ib

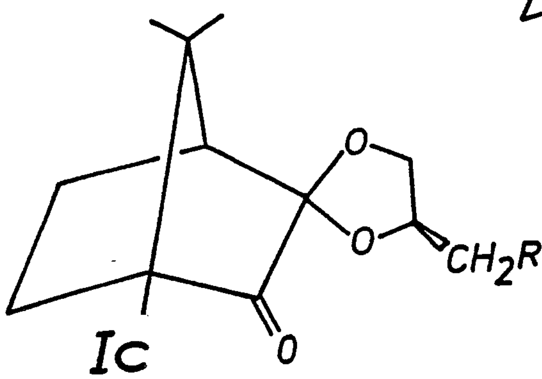

Ic

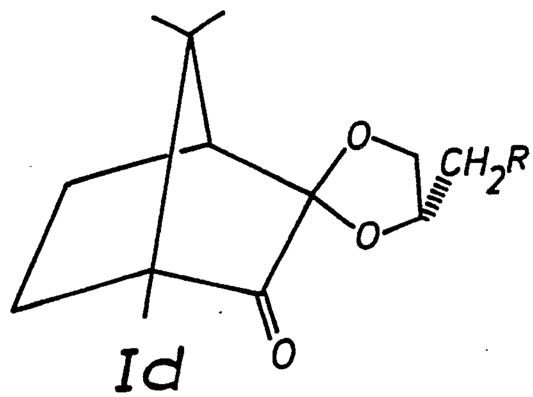

Id

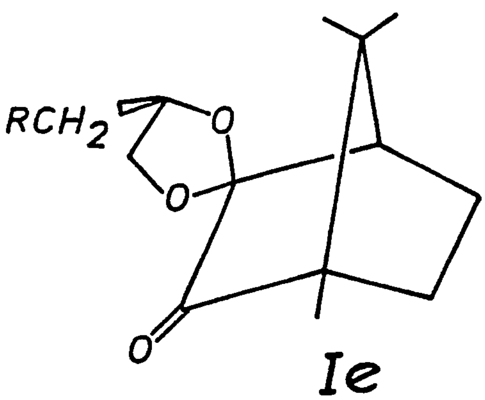

Ie

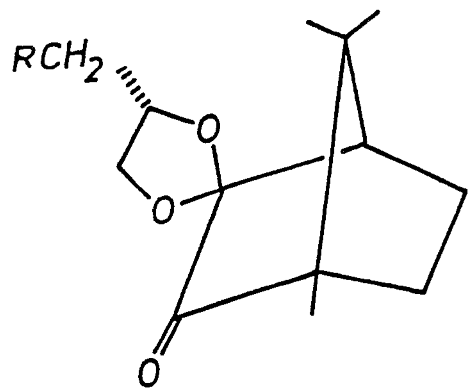

If

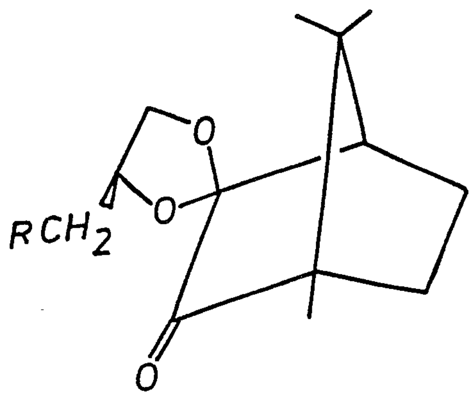

Ig

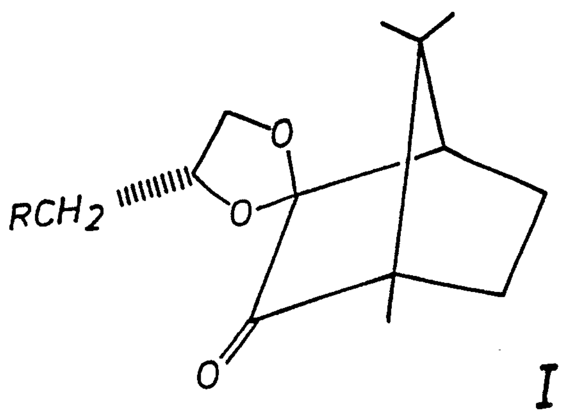

Ih